Europäisches Patentamt

European Patent Office

Office européen des brevets

(11) Publication number: **0 215 536**
**A2**

# EUROPEAN PATENT APPLICATION

(21) Application number: **86300617.7**

(22) Date of filing: **30.01.86**

(51) Int. Cl.⁴: **B01L 3/02**

(30) Priority: **13.08.85 US 765200**

(43) Date of publication of application:
**25.03.87 Bulletin 87/13**

(84) Designated Contracting States:
**DE FR GB NL**

(71) Applicant: **COSTAR CORPORATION**
**205 Broadway**
**Cambridge Massachusetts 02139(US)**

(72) Inventor: **Lyman, George F.**
**Rocky Point**
**Cape Porpoise Maine 04014(US)**

(74) Representative: **Read, Matthew Charles**
**Venner Shipley & Co. 368 City Road**
**London EC1V 2QA(GB)**

(54) **Hand-held media transfer tool.**

(57) The hand tool for transferring media from a source to a multi-well dish includes a base adapted to be selectively placed over the source container and multi-well dish. A transfer plate is mounted in the base to receive media from the source container and discharge it into the multi-well dish. The transfer plate is operated by a piston plate in turn controlled by a cam and lever, and a metering control is provided on the tool handle which cooperates with the lever enabling the operator to select the quantity of media or other liquid transferred and establishes click stops for the lever for the selected quantity.

EP 0 215 536 A2

## Hand-held Media Transfer Tool

This invention relates to equipment used in the laboratory to transfer media to a multi-well culture dish, and more particularly comprises a portable hand tool for that purpose. In copending US applications Serial Nos. 449,775 and 512,722 filed December 14, 1982 and July 11, 1983, respectively (both entitled "Culture Media Transfer Assembly") and having a common assignee with this application, machines are disclosed which function essentially as multi-channel pipetters capable of simultaneously depositing media into all the wells of a multi-well culture plate such as a 96-well tissue culture dish, without cross contamination between the several wells in a single dish or between successive dishes filled by the machine. The hand-held tool of this invention performs many of the same functions as those machines, but is much less expensive, portable, and can be used conveniently in virtually any location.

A typical use of this invention is to transfer cells growing in one media in a multi-well culture dish to a second multi-well dish containing fresh media so as to lessen the cell concentration in each well or to supplement the nutrients available to the growing cells. Another typical use of the hand tool of this invention is to load the wells of a multi-well culture dish with fresh media. In the first example of use of the invention, cells typically growing in a 96-well tissue culture dish may be withdrawn and deposited in a second 96-well dish whose wells contain fresh media. In the second example given, the media may typically be drawn from a reservoir into a transfer plate held by the hand tool and thereafter be simultaneously deposited in the wells of the multi-well tissue culture dish.

One important object of this invention is to provide a relatively inexpensive device for carrying out the transfers.

Another object of this invention is to provide a lightweight, easily carried, hand tool which may be taken any place in a laboratory for transferring media or other material from a source to one or a number of multi-well dishes.

Another object of this invention is to provide a wholly portable 96 channel pipetter capable of simultaneously transferring media to all the wells of any size multi-well dish (to a maximum of 96 wells) without any opportunity for cross contamination from one well to another or from one dish to another.

Another object of this invention is to provide a portable multi-well pipetter which is very accurate in dispensing selecting quantities of liquid.

To accomplish these and other objects, the hand tool of this invention utilizes a transfer plate having a plurality of separate chambers covered on the top by a flexible diaphragm and each open at the bottom. By changing the tool format, it may be useful with dishes having other, larger, numbers of wells, for example, 150 wells. The diaphragm may be stretched so as to extend into the chambers to selectively either discharge the contents of the chambers or draw media or other liquid into them by creating a partial vacuum in the chambers. The hand tool itself has a base which is shaped so as to be selectively placed over the source container of the liquid or over the multi-well dish to receive it. A support in the base above its bottom is oriented to carry the transfer plate in alignment with the multi-well dish or reservoir disposed beneath. A piston plate is movable in the base above the support and has a plurality of pistons for engaging the diaphragm and pushing it into the respective chambers of the transfer plate. A handle extends upwardly from the base, and an actuating lever is mounted on the handle and is connected to the piston plate to move it. A cam and detent cooperate with the lever so as to provide means for adjustably setting the throw of the lever to meter the quantity of liquid drawn into and expelled from the transfer plate.

The hand held tool is lightweight and is merely placed over the source of liquid or the multi-well dish into which the liquid is to be deposited. Because it is small and lightweight, it may be freely carried about the laboratory to wherever the work may conveniently be performed. The tool has few moving parts, is rugged, and yet is capable of very accurate metering of the transferred liquid.

These and other objects and features of this invention will be better understood and appreciated from the following detailed description of embodiments thereof, selected for purposes of illustration and shown in the accompanying drawings.

## BRIEF FIGURE DESCRIPTION

FIG. 1 is a cross-sectional elevation view of one embodiment of the hand tool of this invention shown with a 96 well culture dish in its base and with its piston plate fully elevated;

FIGS. 2 and 3 are cross-sectional views similar to FIG. 1 but showing the piston plate in a "start" position with a 96 well dish and a second position with a reservoir in the base, respectively.

FIG. 4 is an enlarged fragmentary cross-sectional view of the actuating lever of the invention;

FIG. 5 is a fragmentary cross-sectional view of the upper end of the handle along with the metering control of the invention;

FIG. 6 is a fragmentary rear view of the handle showing the calibrated metering scale on the handle;

FIG. 7 is a perspective view of the base of the hand tool and showing the opening for the transfer plate;

FIG. 8 is an exploded view of the transfer plate;

FIG. 9 is a fragmentary cross-sectional view of the edge of the cover of the transfer plate taken along section line 9-9 in FIG. 8;

FIG. 10 is a fragmentary vertical cross-sectional view of the transfer plate;

FIG. 11 is a fragmentary bottom plan view of the transfer plate;

FIGS. 12-17 are diagrammatic views showing the manner in which the transfer plate functions when operated by the hand tool of this invention;

FIG. 18 is a perspective view of a typical 96 well culture dish;

FIG. 19 is a front cross-sectional elevation view of the preferred embodiment of transfer assembly;

FIG. 20 is a cross sectional view of the preferred assembly taken along section line 20-20 in FIG. 19;

FIG. 21 is a fragmentary front view of the base of the assembly shown in FIGS. 19 and 20;

FIG. 22 is a perspective view of the door shown in FIG. 21; and

FIg. 23 is a fragmentary elevation view of the preferred embodiment taken along section line 23-23 of FIG. 19.

## DETAILED DESCRIPTION

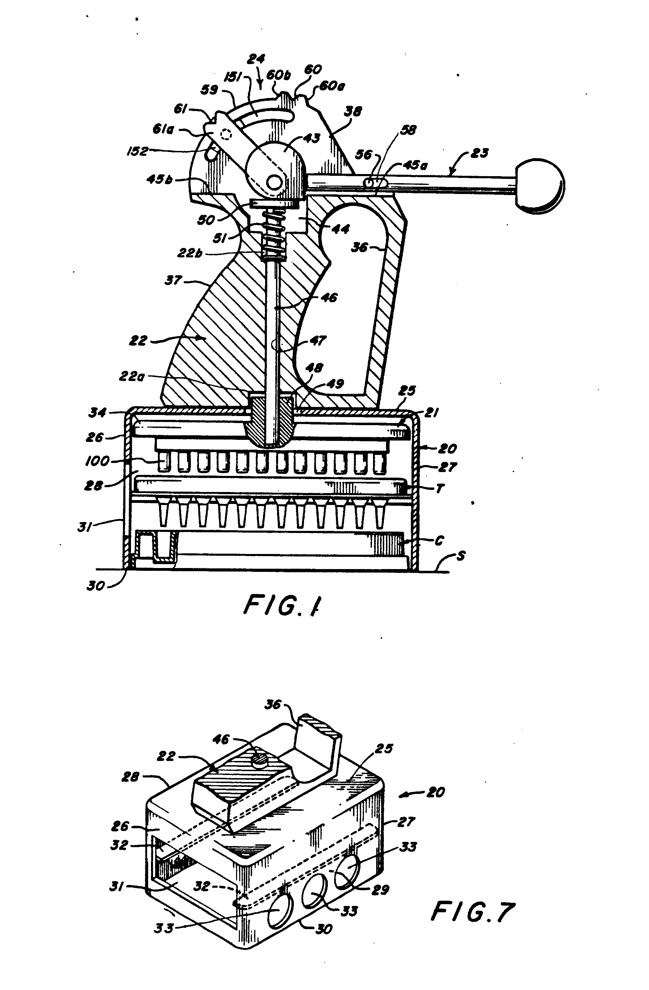

The hand held transfer assembly shown in FIGS. 1 to 7 is used to transfer liquid such as culture media from a source to a multi-well dish which typically may be a 96-well tissue culture plate. The source itself may be a multi-well plate or it may be a reservoir of virgin media. One common application of the device is to dilute tissue cultures by removing cultured media from one multi-well dish and depositing the media in another multi-well dish which contains some virgin material. The hand held tool of this invention enables the transfer to be conveniently, accurately and inexpensively made anyplace in the laboratory.

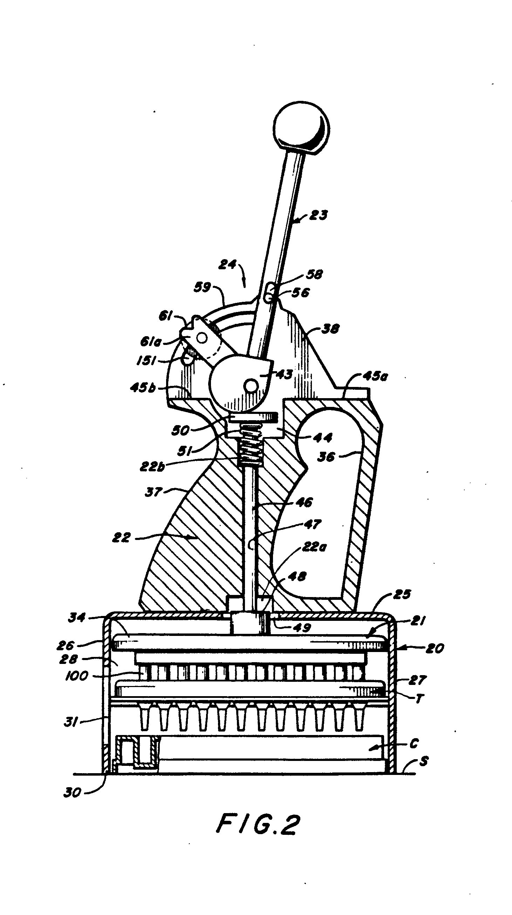

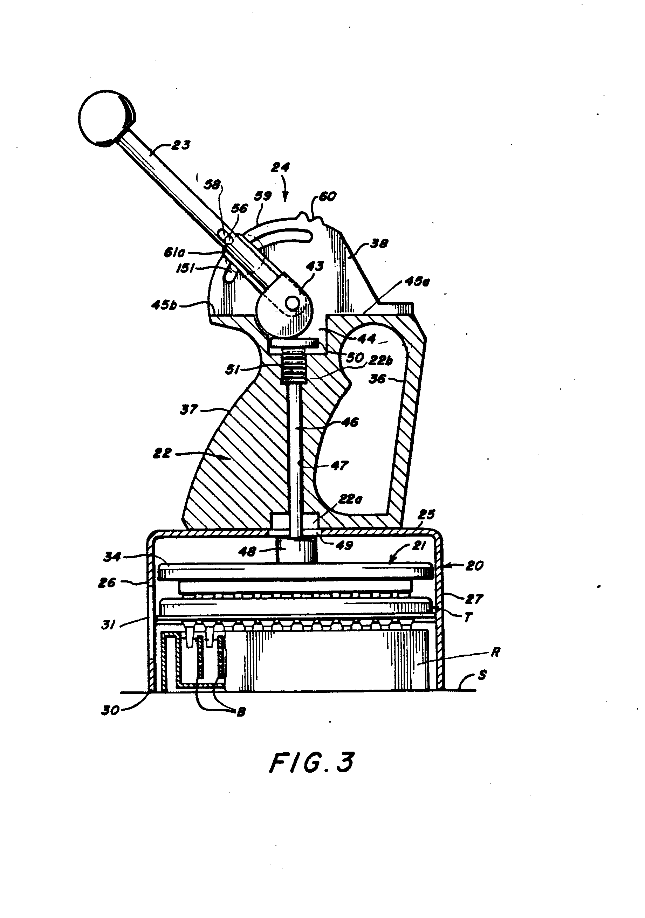

The hand held metering tool includes a base 20 open at the bottom so that it may be placed over the source or receiving receptacle of the liquid to be transferred by the tool. In FIGS. 1 and 2, a 96-well culture dish C is suggested supported on a surface S on which base 20 also rests. In FIG. 3, the base is shown placed over a reservoir R. The base supports a transfer plate T above the culture dish C and reservoir.

A piston plate 21 is mounted in base 20 above the transfer plate T and acts upon the transfer plate causing it to either draw in or expel the liquid to be transferred. A handle 22 is connected to the base 20 and in turn carries a control lever 23 for raising and lowering the piston plate 21. A metering control 24 is also provided in the handle for controlling the throw of the lever.

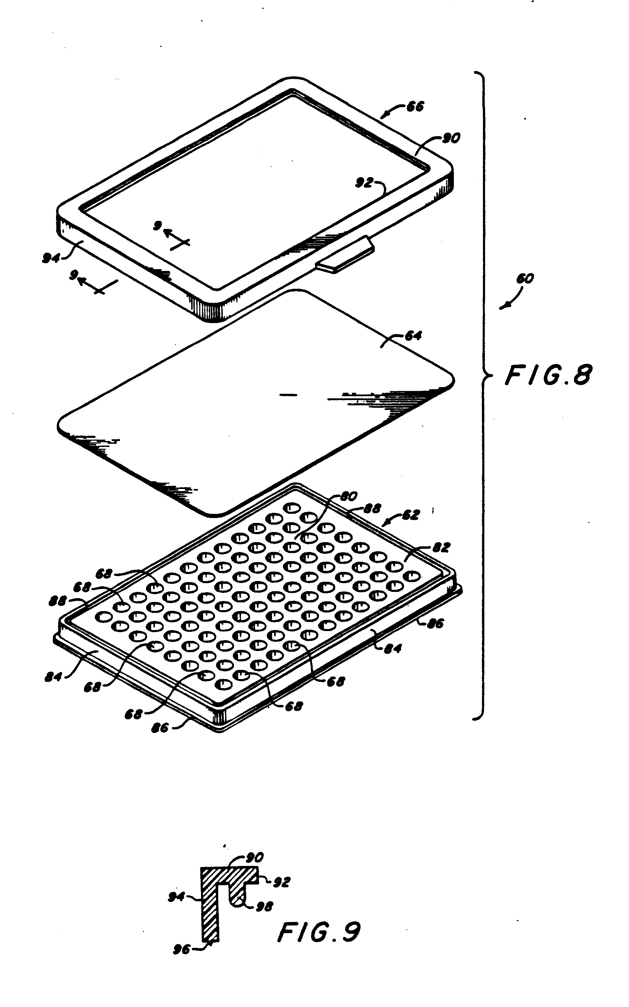

Before describing further details and the operation of the tool and the way it may be used in the laboratory, the transfer plate T used with the machine will be described, as the functions of the tool and the transfer plate are interrelated. The transfer plate is described in detail in copending applications Serial Nos. 449,775 and 512,722, supra. The transfer plate T shown in FIGS. 8 to 11 includes a base 62, membrane 64 and cover 66. Base 62, which is designed to be molded as a unitary structure of inexpensive plastic material such as high density styrene, includes an array of funnel-shaped chambers 68 arranged in eight parallel rows of twelve chambers each. The corresponding chambers in each row in turn form their own rows perpendicular to the twelve chamber rows. The cylindrical side wall 70 of each chamber 68 has a slight downward draft to facilitate stripping of the base from the molds during manufacture. The bottom wall 72 of each chamber is in the shape of an inverted cone and has a central opening 74 communicating with a downwardly converging stem 76 that serves as the intake and discharge passage for the chamber 68. The tops 78 of the several wells 68 are coplanar with the top panel 80 of the base. The periphery of panel 80 defines a horizontal flange 82 about the array of chambers 68, and the panel is surrounded by a downturned skirt 84 having an outwardly turned lip 86 at its bottom. Formed in the periphery of the panel 80 in flange 82 is a groove 88 whose function is described below. The base 62 is stiffened by short ribs 89 on the bottom of panel 80 (see particularly FIG. 11) that extend from the skirt 84 to the side walls 70 of the chambers.

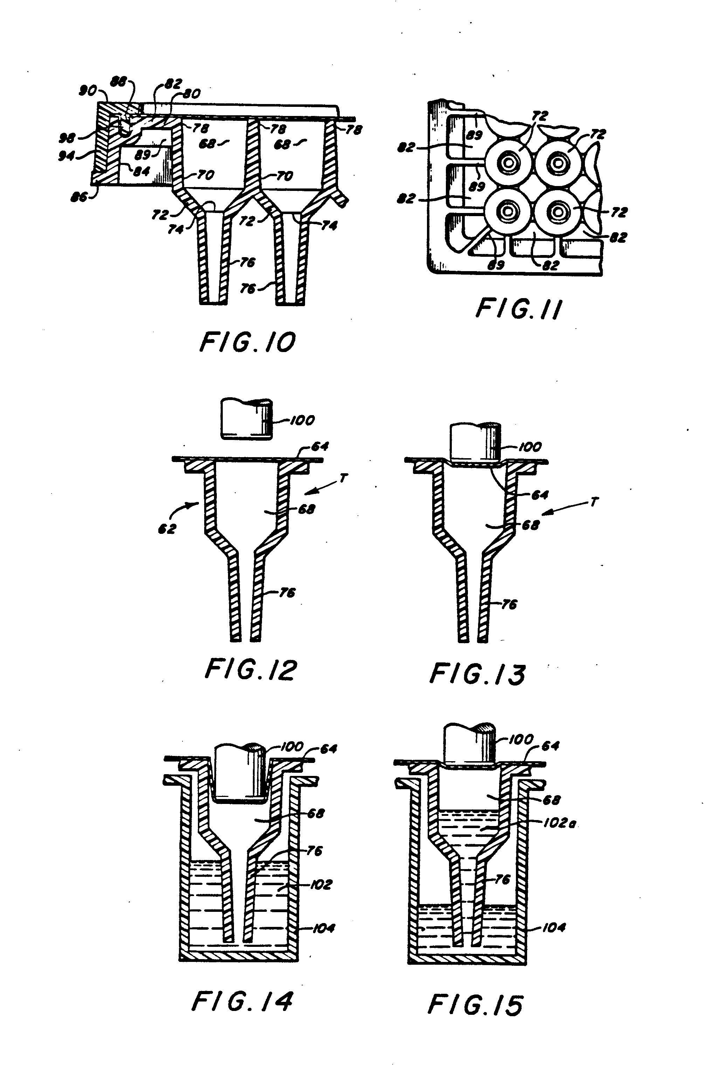

Cover 66 of the transfer plate, which may be made of the same material as the base 62, has a top wall 90 with a large rectangular central opening 92 so that the cover has a frame-like configuration sized to overlie the flange 82 and groove 88 of the base. A rim 94 extends downwardly from the periphery of the wall 90 and is preferably provided with a bead 96 at its bottom (see FIG. 9) which may be ultrasonically welded to the top of lip 86 of base 62 when the transfer plate is assembled. Just inwardly of rim 94 and depending downwardly from the bottom of top wall 90 of cover 66 is a tongue 98 which is sized to fit within groove 88 in panel 80 of the base. When the cover is being assembled on the base, tongue 98 automatically aligns itself with the groove 88 so that the parts may be assembled in the manner shown in FIG. 10.

Diaphragm 64 which typically may be made of latex rubber sheet material is stretchable in two directions and is cut so as to conform in the unstretched condition quite accurately to the plan of top wall 80 of base 62 so as to fully span the groove 88 about the four sides of the base. Consequently, when the diaphragm 64 is loosely laid on the top wall 80 of the base 62 and the cover 66 is applied, the tongue 98 stretches the diaphragm into the groove and holds the diaphragm firmly against the top wall 80 and close the top of each of the chambers 68. The cover and base are ultrasonically welded together along the bottom of the rim 94 and the upper surface of the lip 86 of the cover and base, respectively, at bead 96.

The manner in which the transfer plate 60 operates to remove liquid from one container and deposit it in another is diagramatically illustrated in FIGS. 12-17. In these figures, a single transfer plate chamber 68 is shown. In FIG. 12, base 62 with its chamber 68 and stem 76 are shown covered by diaphragm 64, and disposed above the diaphragm is a piston 100. The chamber 68 is empty, and no pressure is being exerted on the diaphragm 64 by the piston.

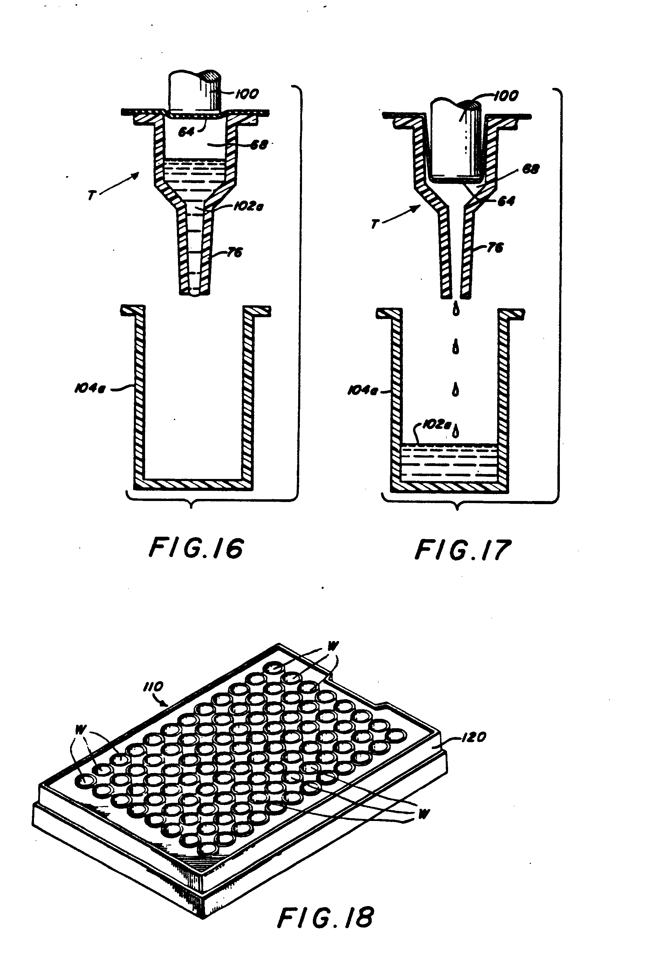

In FIG. 13 the piston 100 is shown to project slightly into chamber 68, and the stretched diaphragm forms a seal about the top of the chamber. The next step in the process is to place the stem 76 of the chamber in the liquid 102 in the transferor container represented by well 104 and move piston 100 further into the chamber as in FIG. 14. If the piston 100 is thereafter partially withdrawn from chamber 68, pressure in the chamber is reduced so as to cause the transfer plate to draw a portion of the liquid 102 into the chamber from well 104 as is suggested as 102a in FIG. 15. Piston 100 does extend, however, a short distance into the chamber 68 as in FIG. 13 so as to maintain the air tight seal between the diaphragm and the edge of the top 78 of the chamber.

The partially filled chamber 68 is then removed from the liquid 102 in the well 104, and the chamber 68 retains the column of fluid 102a because of the difference in pressure above and below the column. FIG. 16 suggests that an empty transferee container 104a is positioned beneath the transfer plate 60 to receive the liquid column 102a. To discharge the column of liquid 102a into the well 104a, the piston 100 is rapidly driven to a lowermost position in chamber 68 as shown in FIG. 17. The rapid increase of pressure applied to the top of the liquid will cause the liquid to flow from the chamber 68 to well 104a as suggested.

The hand held tool shown in FIGS. 1 to 7 is designed to carry out the sequence of steps with the transfer plate T shown in FIGS. 12 to 17. And it is calibrated by means of the metering control 24 so as to allow the tool to transfer precisely measured quantities of liquid from the transferor source to the transferee container and provide it with accurate repeatability.

A typical multi-well plate which may contain the source of the liquid media and/or serve as the plate to receive the liquid is shown in FIG. 18. The configuration of that plate and others that may be used with the tool form no part of the present invention, and the plate of FIG. 18 is described only briefly. The plate includes eight rows of twelve wells W each, and the plate has a surrounding rim 120 which supports it on a horizontal surface. The bottoms of the wells W are closed, while the tops of the wells are open so as to afford ready access to them. The general format of the wells W in the plate has been standardized by the industry so that the centres of the wells are an established distance apart and the overall plate size is determined. Typically, a cluster dish of the type shown in FIG. 18 is used to grow separate tissue cultures for experimental or test purposes. The multi-well dish provides a convenient means of keeping together a number of correlated tests while maintaining their separation by virtue of their separate independent wells. During normal testing procedures, it is frequently desired to dilute the tissue cultures, and this may be accomplished by removing a part of the contents of each well and depositing the material in another multi-well dish with additional fresh media. The tool of this invention along with its transfer plate is ideally suited for this operation.

Base 20 of the portable tool shown in FIGS. 1-7 may be made of aluminum or molded of an autoclavable plastic material such as epoxy or other material and has a top wall 25 and side walls 26, 27, 28 and 29. The lower edges 30 of the side walls are flat and are designed to rest on the surface S of the table on which the multi-well cluster dish C or other reservoir R rests. The plan dimensions of the base 20 are such that it will just fit over the dish C or reservoir R.

As shown in FIGS. 1-3 and 7, side wall 26 is provided with an opening 31 across substantially its full width so as to allow a transfer plate T to be inserted into the base 20 on top of the side rails 32 carried on the walls 28 and 29. Rails 32 support the downturned lips 86 of the skirt 84 of the transfer plate with the separate chambers 68 and their stems 76 extending downwardly in the direction of the dish C within base 20. The rails are designed to hold the transfer plate firmly in position and prevent it from bowing under the load applied to it by the piston plate 21, as is described more fully below.

A number of ports 33 in side wall 29 are provided so as to enable the user of the tool to view its operation. The ports 33 are disposed beneath the rails 32 so as to enable the user to visually confirm that the funnel-shaped stems 76 are precisely aligned with the wells in the dish C from or to which liquid is to be transferred. If the base is made of transparent material, obviously the windows may be eliminated.

The piston plate 21, which is made of a cast epoxy or other similar material capable of being autoclaved and which is very rigid, includes a heavy head 34 from which the individual pistons 100 depend. The pistons 100 are arranged in precisely the same configuration as the wells W in the 96-well plate shown in FIG. 18. Both their spacing, center to center, and their number are the same.

Handle 22, which is also made of plastic, is screwed or otherwise detatachably connected to top wall 25 of base 20, and is pistol-grip in shape with an opening 36 through which the fingers may extend for ease of handling. The back edge 37 of the handle is contoured so as to fit the palm of the hand. Extending vertically downward in the handle from the top is a slot 38 between the parallel walls 39 and 40 which are part of the handle. A horizontal shaft 41 mounted in bearings 42 in walls 39 and 40 spans the slot 38 and supports an eccentric cam 43 rotatable with the shaft. A shallow well 44 extends downwardly from the bottom of slot 38 and partially houses cam 43. The control lever 23 extends out slot 38 and is connected by dowel 43a to the cam. The bottom surfaces 45a and 45b of the slot serve as stops for lever 23 to limit rotation of the cam about the axis of shaft 41. In FIG. 1 lever 23 is shown in one extreme position established by surface 45a.

Cam 43 operates stem 46 that extends through opening 47 in handle 22 and which is connected to the head 34 of the piston plate 21 by means of collar 48. The collar 48 extends upwardly through opening 49 in top wall 25 of the base and into the cavity 22a in the handle. The collar 48 and stem 46 are secured together so that the piston plate 21 moves vertically in base 20 as stem 46 moves in response to rotation of cam 43. The top end of stem 46 carries a shoe 50 against which cam 43 operates. Stem 46 is biased to the elevated position shown in FIG. 1 by spring 51 which surrounds the upper end of the shaft, sits in a counterbore 22b at the bottom of well 44, and bears against the bottom of shoe 50.

It will be appreciated that when the cam 43 is in the position shown in FIG. 1, piston plate 21 is in the elevated position, and as the cam 43 rotates from the position of FIG. 1 to the positions of FIGS. 2 and 3, the piston plate 21 is moved downwardly in the direction of the transfer plate T. Continued rotation of the cam by the lever 23 to a position wherein the lever contacted surface 45b moves the piston plate 21 even further downwardly so that its pistons 100 extended further into the chambers 68 of the transfer plate.

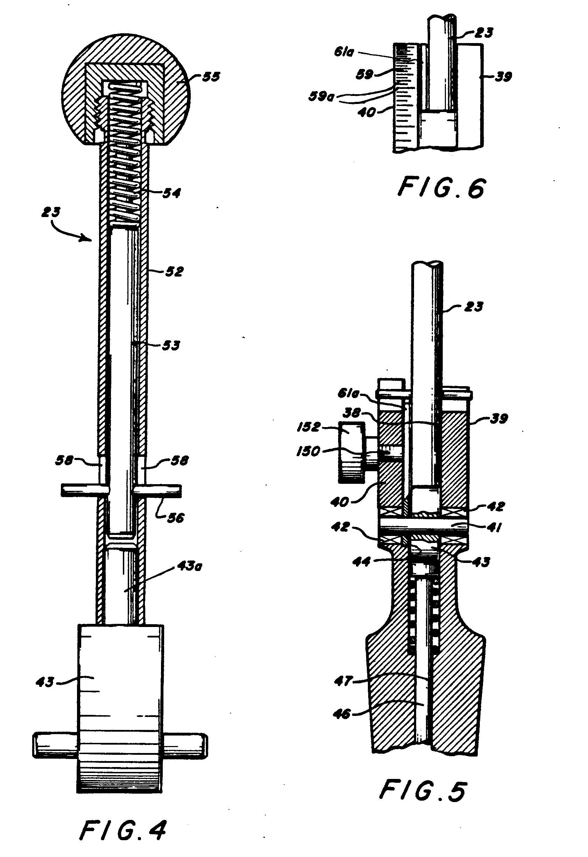

Lever 23 which through the cam and stem 46 operates the piston plate 21 is provided with a metering control assembly that provides detent stops for the lever. As shown in FIG. 4, the lever includes a hollow sleeve 52 containing a slide 53 that moves axially in it but which is biased in the direction of cam 43 by spring 54 trapped in sleeve 52 between the top of slide 53 and knob 55. A cross pin 56 is carried by slide 53 and extends out the slots 58 formed in the sleeve. The ends of the slots 58 thereby define the limits of travel of the slide 53. Spring 54 biases the slide to the position shown in FIG. 4, but the slide may be moved to an elevated position within the sleeve against the action of spring 54 by the detents formed at the arcuate edges 59 of slot 38 at the top of the handle.

In FIG. 1, a first detent 60 is shown formed in the arcuate edges 59 of walls 39 and 40 which define the sides of slot 38. Ramps 60a and 60b are provided at the sides of detent 60 so as to enable the pin 56 to ride up to and enter detent 60 so as to establish a first click stop for lever 23. A second detent 61 is provided at the periphery of slot 38 at the end of finger 61a pivotally supported on shaft 41 as shown in FIGS. 1 and 5. Finger 61a carries a threaded stud 150 which extends through arcuate slot 151 formed in wall 40. A knob 152 is threaded onto the stud on the outside of wall 40 and may be tightened to draw finger 61a firmly against wall 40 so as to hold the finger in any selected position, limited only by the extremes of the arcuate slot 151. The setting selected for the finger 61a will be determined by the quantity of liquid to be drawn into and dispensed from the chambers of the transfer plate T during one cycle of operation of the tool. As is shown in FIG. 6, the arcuate edge 59 of wall 40 is calibrated at 59a so as to enable the user to preset the second detent 61 at the selected volume. A reference line or pointer may be carried by the finger to cooperate with the calibrations for more accurate settings.

The tool of FIGS. 1-7 operates as follows:

Assume that it is to be used to deposit virgin media to be taken from a reservoir into a number of empty 96-well culture dishes. Lever 23 is moved to the position shown in FIG. 1 and the operator slides a transfer plate T onto the rails 32 with the diaphragm 64 of the transfer plate opposite the pistons 100 and with the individual pistons axially aligned with the chambers 68 in the transfer plate as in FIG. 12. After the transfer plate T is inserted in place, the lever 23 may be pivotted to the position shown in FIG. 2 wherein the pin 56 sits in the detent 60 which establishes the first click stop for the piston plate 21. As the lever 52 is swung from the position shown in FIG. 1 to that of FIG. 2, the pin 56 engages the ramp 60a on the side of the detent, rides up to the top of the detent against the bias of spring 54 which continuously urges pin 56 against the bottom of the slots 58, and the pin 56 ultimately drops into the detent under the urging of spring 54. With the lever 23 in the position shown in FIG. 2, the pistons 100 slightly deflect the diaphragm 64 of the transfer plate so as to seal the tops of the chamber 68 in the manner shown in FIG. 13. The open bottoms of the chambers, however, are not disposed in the media. The operator then lifts the tool by grasping the handle 22 and places the base 20 over the reservoir R as suggested in FIG. 3 so that the funnel-shaped stems 76 of the chambers 68 of the transfer plate extend into the media in the reservoir. The reservoir R shown forms no part of the present invention, but it will be noted that it does include baffles B which prevent the media from sloshing about. The baffles do, however, have holes so as to allow the media to seek a uniform level throughout the reservoir. Before drawing liquid from the reservoir into the chambers 68 of the transfer plate, the operator should adjust the finger 61a with reference to the calibrations 59a to establish the quantity of media to be drawn in the chambers 68. This is done by loosening the knob 152 and pivotting the finger 61a on shaft 41 to the selected position and thereafter again tightening the knob 152 so as to hold the finger firmly in place. This adjustment may, of course, be carried out at the very beginning of the operation or at any time prior to moving the lever from the position shown in FIG. 2 to the next position for filling the transfer plate.

Having set the adjustable detent for the selected volume, the operator next moves the lever 23 to the position shown in FIG. 3 wherein pin 56 engages the second click stop established by the adjustable detent 61. This will cause the pistons 100 to assume a position in the chambers 68 as suggested in FIG. 14. The operator next returns the lever 23 to the position of FIG. 2 established by detent 60, which creates a partial vacuum in the chamber 68 to draw media into it in the manner of FIG. 15. With the media held in the chambers of the transfer plate, the operator then lifts the tool manually off the reservoir R and places it over the empty 96-well culture dish C into which the media is to be deposited as in FIGS. 2 and 16. In this position, the operator then moves the lever from the position shown in FIG. 2 over and beyond the second adjustable detent 61 so that the lever 23 engages stop 45b to drive all of the liquid from the chambers 68 into the wells of the culture dishes. The piston 100 in FIG. 16 is shown in its lowermost position wherein it fully extends the diaphragm 64 to empty the transfer plate. With the contents of the transfer plate discharged, the operator may return the lever 23 to the position shown in FIG. 2 - (or FIG. 1) and the sequence of steps described may be repeated by again drawing media from the reservoir R into the transfer plate and thereafter discharging the media from the transfer plate into the next 96-well dish.

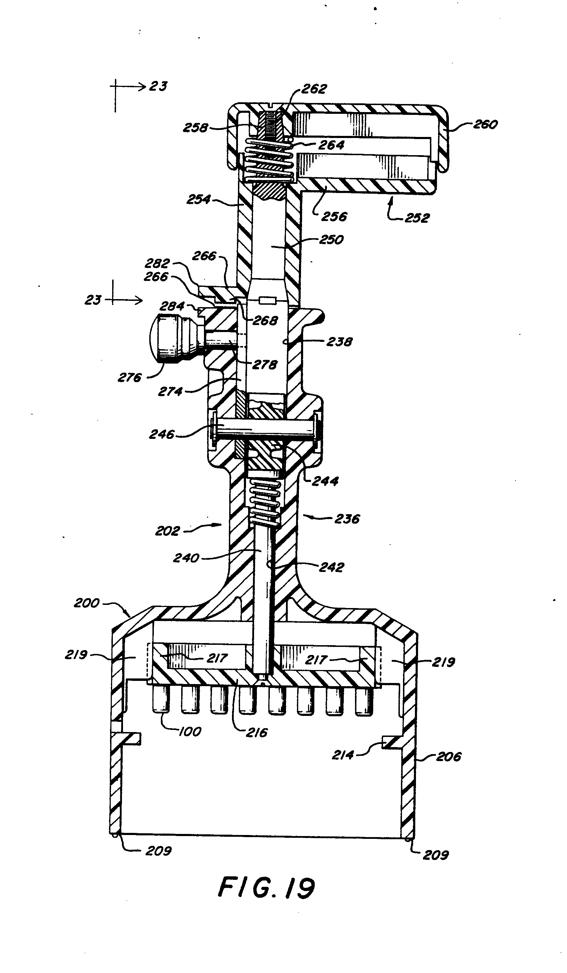

In FIGS. 19-23, the preferred embodiment of this invention is shown. The hand held unit of this embodiment includes three principal improvements over that shown in FIGS. 1-7. First, the preferred embodiment includes means for ensuring that the transfer plate used with the tool is precisely aligned with the piston plate so as to prevent breakage. Second, the actuating mechanism has been modified so that the detent may be manually retracted to avoid shaking of the assembly which may cause spillage of the liquid being transferred. And third, a lens is incorporated into the assembly, through which the calibrations on the handle may be viewed to assist in controlling the volumes of liquid transferred. These improvements are described in detail below.

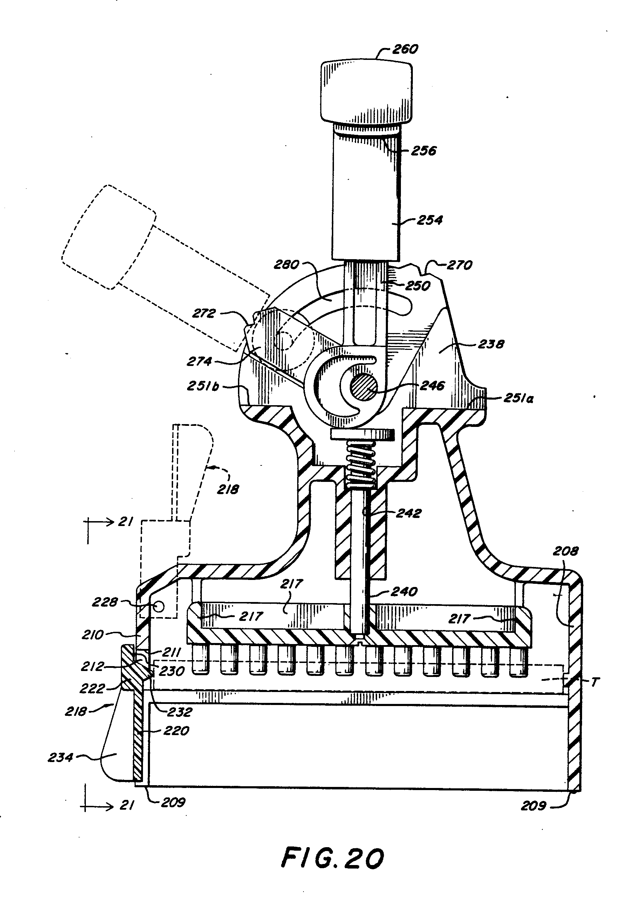

As shown in FIGS. 19 and 20, the improved tool includes a base 200 and handle 202 integrally molded as a unitary structure from an autoclavable transparent plastic material and which together comprise the body of the device. Base 200 has a pair of parallel vertical side walls 204 and 206 and a vertical rear wall 208 whose bottom edges 209 are coplanar and intended to support the tool on a horizontal table or other surface. The front of the base has a short front wall 210 with a bottom edge 211 that lies well above the edges 209 to define an opening 212 in the base that provides access to the interior. A pair of rails 214 are integrally molded with the side walls 204 and 206 of the base for supporting the transfer plate used with the tool, as in the first described embodiment. The side walls 204 and 206 are spaced just far enough apart so as to allow the transfer plate to enter the base between them and rest on rails 214.

Piston plate 216 which is shown made of a plastic material carries the pistons 100 and lies within the base 200. The pistons 100 are arranged in eight rows of twelve pistons each, just as in the first embodiment. The plate 216 has a peripheral upwardly extending side wall 217 that cooperates with guide flanges 219 in the base to confine the movement of the piston plate to a precise vertical path. The side walls 204 and 206 along with rails 214 and the guide flanges 219 ensure that the eight rows of wells in the transfer plate on the rails 214 are aligned with the eight rows of pistons 100. A door 218 is supported on the base 200 over the opening 212 and serves to align the 12 transverse rows of wells in the transfer plate with the transverse rows of pistons 100 carried by the piston plate 216. When the door 218 is closed, the transfer plate is automatically pushed fully into the base 200 on the rails 214 so as to ensure that the plate is centered both longitudinally and transversely with respect to the piston plate.

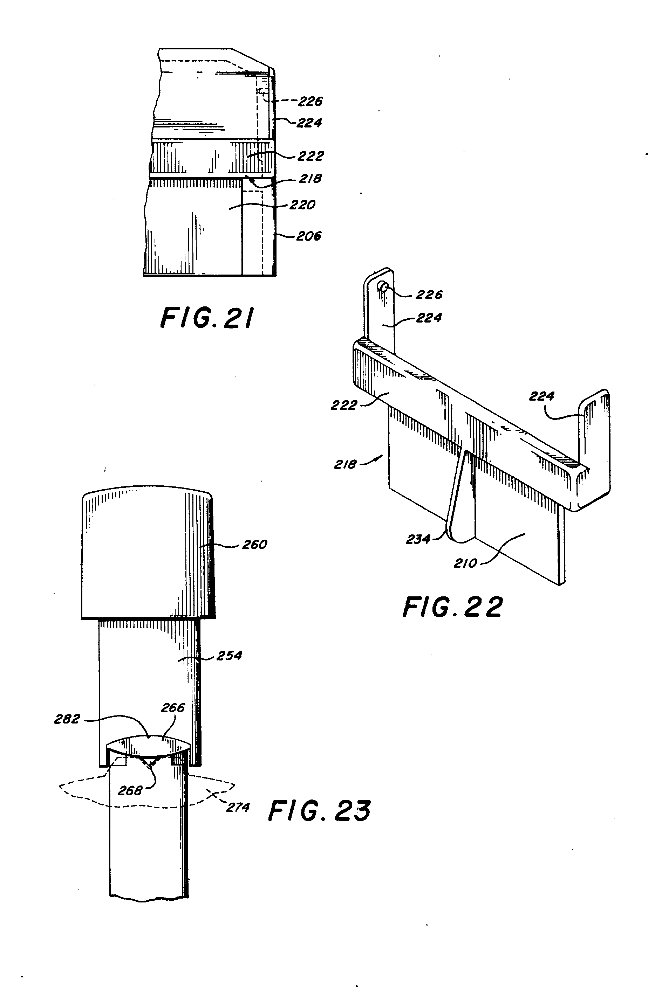

The door 218 as shown in FIGS. 20 and 21 includes a vertical panel 220 carried on a horizontal bar 222 which in turn is suspended from a pair of vertical arms 224 that lie outside the sidewalls 204 and 206 of the base adjacent the front wall 210. Pivot pins 226 are integrally molded with the arms 224 adjacent their upper edges. The pivot pins 226 extend into openings 228 in the sidewalls 204 and 206 to support the door 218 in place. The inner face 230 of horizontal bar 222 carries a pair of fingers 232 positioned to engage the outer end of the rim 94 of transfer plate T disposed on the rails 214 when the door is closed (see FIG. 20) so as to push the transfer plate fully onto the rails and against the inner surface of the real wall 208 to achieve alignment of the transfer plate with the pistons 100.

Door 218 also carries a handle 234 to facilitate swinging the door from the closed position shown in full line to the elevated or opened position shown in broken lines in FIG. 20. The door is designed to swing through an arc of 180°, and it will remain in the open or raised position when the center of gravity of the door passes over the axis of the pivot pins 226.

As described above and as is evident in the drawings, in the preferred embodiment shown in FIGS. 19 and 20 the handle 202 of the tool is integrally molded with the base 200. The handle is provided with a vertical slot 238 at its upper end which corresponds to the slot 38 in the first-described embodiment. The handle, however, does not have a finger opening which corresponds to the opening 36 in the first embodiment. Rather, the handle 202 is hollow and relatively squat as compared to the earlier embodiment. The handle has a longitudinal opening 242 that carries stem 240 which moves vertically in the opening and is actuated by cam 244 mounted on shaft 246 that extends across the slot 238. The cam 244 is shown carried on the lower end of lever 250.

Unlike the first described embodiment, in the preferred embodiment of this invention the detent used to establish the intermediate positions of the lever 250 is manually retracted by actuation of the handle grip 252. The extreme positions of the lever are established by surfaces 251a and 251b that correspond to surfaces 45a and 45b in the first embodiment. The grip which is mounted on the lever 250 includes a sleeve 254 slideable axially on the lever and which carries an engagement arm 256 that the fingers engage when the operator grasps the handle grip. The free end 258 of lever 250 carries a cap 260, and the two are secured together by flat headed screw 262. A spring 264 surrounds the upper end of lever 250 and bears against the inner surface of the cap 260 and the end of sleeve 254 so as to bias the sleeve in the direction of cam 244.

Sleeve 254 carries a flange 266 having a detent 268 on its bottom, which is shaped to engage the detent notches 270 and 272 which, respectively, are at the top of handle 236 and on the free end of finger 274 and correspond to the notches 60 and 61 in the first described embodiment. As in the first embodiment, the position of finger 274 is controlled by a knob 276 threaded onto the end of shaft 278 secured to finger 274 and slideable in the arcuate slot 280.

In the first embodiment, pin 56 carried by lever 23 moves within the slot 58 in the lever 23 as it rides up and down the ramps on the sides of the detent notches 60 and 61. In the preferred embodiment now described, detent 268 is retracted manually when the operator squeezes the handle grip 252 which elevates arm 256 and raises the sleeve 254 on lever 250 against the action of spring 264. When the detent 268 is aligned with either of the detent notches, the operator may release the arm 256 so as to allow the sleeve 254 to move under the influence of the spring 264 and cause the detent 268 to engage the notches. This arrangement avoids any jarring of the tool which may occur in the first embodiment as the pin 56 snaps in and out of the detent notches 60 and 61. Thus, the preferred embodiment provides a smoother operation of the device and eliminates any possible jarring of the instrument which may cause unintended spilling of liquid from the multi-well dishes or reservoirs with which the tool is used.

Yet another improvement provided in the preferred embodiment is lens 282 which may be formed as an integral part of sleeve 254, flange 266 and detent 268. As shown in FIGS. 19 and 23, lens 282 overlies the surface 284 at the top of handle 236 at the margin of slot 238, and which carries the calibration markings 286. When the calibration markings on the surface 284 are viewed through the lens, they are magnified by it and therefore are easier to read. This will assist in the positioning of the finger 274 particularly if the finger 274 is moved with the lever 250 by virtue of the engagement of the detent 268 with its detent notch 272. This obviously may be accomplished by loosening the knob 276 to allow the finger 27 to move freely on shaft 246 and by moving the lever 250 without squeezing the handle grip 252.

From the foregoing description it will be appreciated that with each embodiment of this invention the tool is completely isolated from the media by diaphragm 64 of the transfer plate and consequently it may be used to perform a series of transfers without cleaning. Successive transfers of virgin media from a reservoir to empty 96-well dishes may be carried out without changing the transfer plate. On the other hand, if transfers are to be made involving cultures, cross contamination may be avoided merely by changing the transfer plate, which is disposable. The metering control allows successive transfers made by the tool to be both accurate and constant.

In some procedures where 96-well plates are used, only the inner 60 wells are utilized. This is done because the inner wells are more thermally constant as the ventilation is more even as the plates are moved from one environment to another. When it is desired to utilize only the 60 inner wells, the reservoir used may be of a size just exceeding the plan dimensions of the inner 60 wells of the transfer plate. When this is done, only the inner 60 stems of the transfer plate are positioned within the reservoir, and the outer 36 stems are beyond the reservoir periphery.

It will be appreciated that with the use of the portable tools of this invention, since each piston-diaphragm-chamber member acts independently of the others, media is only transferred from wells containing media and empty wells do not affect the operation of the device. This is a distinct advantage over other systems which generate a single vacuum over all of the wells to remove media from a multi-well plate. In such systems if one or more wells is empty, the system cannot operate because the vacuum will be relieved through the stems which are not submerged in media.

A principal advantage of the present invention is its portability. The tool is very light and can conveniently be transported about the laboratory so that it can be used at any convenient location. With the lever 250 of the preferred embodiment in the upright position in FIG. 20, the overall height of the tool is approximately ... inches, and the tool is approximately 3.5" by 5" in plan. The preferred embodiment weighs only ... pounds. Consequently, it may be conveniently transported from place to place. And the tool may be disassembled for cleaning merely by removing the screws which secure the parts together. All of the parts may be autoclaved.

Having described this invention in detail, those skilled in the art will appreciate that numerous modifications may be made of the invention without departing from its spirit. Therefore, it is not intended to limit the breadth of this invention to the single embodiment illustrated and described. Rather, the scope of the invention is to be determined by the appended claims and their equivalents.

**Claims**

1. A portable hand tool for transferring liquid such as media to a multi-well culture dish and which utilizes a transfer plate having a plurality of separate chambers which receive and discharge media comprising:

a base for alternately receiving a source container of media and a multi-well dish to receive the media;

a support in the base for carrying the transfer plate above the source container and dish;

a piston plate having a number of pistons for entering and moving between selected positions in the chambers of the transfer plate to draw media into the chambers from the source container and discharge the media into the wells of the multi-well dish;

a stem secured to the piston plate for moving the pistons in the chambers and a cam operatively connected to the stem for actuating it;

an actuator for the moving the cam;

a plurality of detent means cooperating with the actuator for establishing stops for the actuator which correspond to selected positions for the pistons in the chambers;

said base having an opening in the side for sliding the transfer plate on and off the support; and

said base carrying a cover at said opening for aligning the transfer plate with the pistons.

2. A hand tool as defined in claim 1 further characterised by

a handle secured to the base and said stem being slidable therein;

said actuator being a lever connected to the cam and mounted on the handle;

and said detent means including a manually retractable flange slidably mounted on the lever for engaging a detent slot on the handle.

3. A hand tool as defined in claim 2 further charaterised by

calibration markings applied to the handle

and a magnifier carried by the lever for facilitating viewing of the calibrations.

4. A portable hand tool for transferring liquid such as media to a multi-well culture dish and which utilizes a transfer plate having a plurality of separate chambers which receive and discharge media comprising

a base for alternately receiving a source container of media and a multi-well dish to receive the media;

a support in the base for carrying the transfer plate above the source container and dish;

a piston plate having a number of pistons for entering and moving between selected positions in the chambers of the transfer plate to draw media into the chambers from the source container and discharge the media into the wells of the multi-well dish;

a handle secured to the base,

a stem slidably mounted in the handle and connected to the piston plate for raising and lowering the plate,

a cam mounted in the handle for controlling the position of the stem and the piston plate,

a slot in the handle and a lever pivotally mounted in the slot and connected to the cam for operating said cam,

a detent flange carried by the lever and at least one detent notch provided on the handle for receiving the flange and establishing a set position for the cam and piston plate, and

manually operated means connected to the detent flange for retracting it from the notch for enabling the cam and the piston plate to move to another position.

5. A hand tool as defined in claim 4 further characterised by

said manually operated means comprising a sleeve slidable on the lever and carrying the flange,

a handle grip carried on the lever and connected to the sleeve for manually retracting the flange so as to prevent the flange from engaging the notch.

6. A hand tool as defined in claim 5 further characterised by

a finger mounted in the slot carrying the notch,

and means for moving the finger to change the set position established by the notch for the cam and piston plate.

7. A hand tool as defined in claim 6 further characterised by

calibration markings on the handle adjacent the path of the flange as the lever moves in the slot,

and a magnifier on the flange for enlarging the calibrations for easier viewing.

FIG. 1

FIG.7

FIG.2

0 215 536

FIG.3

FIG.4

FIG.5

FIG.6

FIG.8

FIG.9

FIG.10

FIG.11

FIG.12

FIG.13

FIG.14

FIG.15

FIG.16

FIG.17

FIG.18

FIG. 19

FIG. 20

FIG.21

FIG.22

FIG.23